# EUROPÄISCHE PATENTANMELDUNG

(11) **EP 3 476 585 A1**
(43) Veröffentlichungstag der Anmeldung: **01.05.2019**
(21) Anmeldenummer: 18199522.6
(22) Anmeldetag: 10.10.2018
(51) Int. Cl.: B31F 1/20, G01N 25/72, G01N 33/34

(54) **MESSSYSTEM FÜR WELLPAPPENMASCHINE**

(30) Priorität: 25.10.2017 DE 102017219064
(71) Anmelder: Texmag GmbH Vertriebsgesellschaft, 8800 Thalwil (CH)
(72) Erfinder: VONDERHEIDEN, Jörg, 22941 Bargteheide (DE)
(74) Vertreter: Peterreins Schley

(57) **Zusammenfassung**

Die vorliegende Erfindung betrifft ein Messsystem (1) zur Verwendung in einer Wellpappenmaschine sowie eine Wellpappenmaschine und ein Verfahren zur Überprüfung des Herstellungsprozesses von Papierverklebungen mit einem derartigen Messsystem. Das erfindungsgemäße Messsystem zur Verwendung in einer Wellpappenmaschine umfasst eine Messeinrichtung (10). Die Messeinrichtung kann dabei elektromagnetische Wellen im Infrarot-Bereich erfassen und ist auf einen Bereich (31) einer Transporteinrichtung (27) der Wellpappenmaschine in Transportrichtung (27) hinter einer Klebestation (22) der Wellpappenmaschine gerichtet.

## Beschreibung

### Technisches Gebiet

Die vorliegende Erfindung betrifft ein Messsystem zur Verwendung in einer Wellpappenmaschine sowie ein Verfahren zur Überprüfung des Herstellungsprozesses von Papierverklebungen.

### Hintergrund

Bei der Produktion von als Materialbahnen hergestellten Erzeugnissen, insbesondere auch Papierverklebungen wie beispielsweise Wellpappenverklebungen, ist eine Beobachtung und/oder automatisierte Qualitätssicherung von hoher Bedeutung, um eine ausreichende Verklebegüte sicherzustellen. Bei der Herstellung einer Wellpappenbahn, beispielsweise einer einseitige Wellpappenbahn aus einer gewellten Papierbahn und einer Deckbahn, also einer flachen Papierbahn, wird ein Klebemittel auf die gewellte Papierbahn und/oder die Deckbahn aufgetragen. Unmittelbar danach werden die gewellte Papierbahn und die Deckbahn zusammengeführt und in Kontakt gebracht, so dass das Klebemittel an den Kontaktstellen eine adhäsive Verbindung zwischen den beiden Bahnen herstellen kann. Für eine erfolgreiche Klebeverbindung ist hier unter anderem die Menge und eine gleichmäßige Verteilung des aufgetragenen Klebemittels ausschlaggebend. Zur Überprüfung einer erfolgreichen Verklebung existieren verschiedene Überwachungssysteme.

Beispielsweise kann die Klebefilmdicke auf einer Klebewalze vor und nach dem Klebeauftrag gemessen werden. Darauffolgend kann indirekt bestimmt werden, ob theoretisch ein ausreichendes Klebevolumen an die zu verklebenden Stelle abgegeben werden konnte. Jedoch lässt dieses System keinen Rückschluss darüber zu, ob sich diese Klebemenge auch wirklich an der zu verklebenden Stelle befindet, geschweige denn, ob die Klebeverbindung zweier Bahnen erfolgreich zustande gekommen ist.

Andere Ansätze verfolgen das Messen der Dicke des Klebefilms nach dem Auftragen auf die gewellte Papierbahn. Hierdurch kann zwar die effektiv aufgetragene Klebemittelmenge bestimmt werden, allerdings lässt dies immer noch keinen Rückschluss über eine erfolgreiche Klebeverbindung nach dem Klebeauftrag und dem Inkontaktbringen zweier Papierbahnen zu. Aufgabe der vorliegenden Erfindung ist es ein Messsystem zur Beurteilung der Verklebegüte von Papierverklebungen, insbesondere zur Beurteilung der Verklebegüte von Wellpappenverklebungen bereitzustellen.

### Zusammenfassung der Erfindung

Die vorliegende Erfindung betrifft ein Messsystem zur Verwendung in einer Wellpappenmaschine sowie ein Verfahren zur Überprüfung des Herstellungsprozesses von Papierverklebungen.

Das erfindungsgemäße Messsystem zur Verwendung in einer Wellpappenmaschine umfasst in einer ersten Variante eine Messeinrichtung. Die Messeinrichtung kann dabei elektromagnetische Wellen im Infrarot-Bereich erfassen und ist auf einen Bereich einer Transporteinrichtung der Wellpappenmaschine in Transportrichtung hinter einer Klebestation der Wellpappenmaschine gerichtet.

Dadurch, dass die Messeinrichtung Infrarotstrahlung erfassen kann, ergibt sich grundsätzlich die Möglichkeit, dass eine Temperatur von Wellpappe erfasst werden kann. Ausgehend von der Temperatur können Rückschlüsse auf die Qualität der Wellpappe, insbesondere auf die Qualität der Verklebung von Wellpappe geschlossen werden. Dies ist gerade deshalb möglich, weil die Messstation auf einen Bereich der Transporteinrichtung in Transportrichtung hinter der Klebestation gerichtet ist. Mit anderen Worten bedeutet dies, dass die Wellpappe nach dem Verkleben, also nach dem Klebemittelauftrag und dem Inkontaktbringen zweier zu verklebender Papierbahnen überprüft werden kann. Das heißt die Wellpappenverklebung wird erst dann überprüft wenn der Klebevorgang abgeschlossen ist und sich das Klebemittel im Aushärtevorgang befindet.

Generell ist hier mit Papierbahn ein Endlosmaterial aus Papier zu verstehen. Dies kann beispielsweise eine gewellte Papierbahn oder eine flache Papierbahn, auch Deckbahn genannt, sein. Einseitige Wellpappe wird beispielsweise aus einer flachen Papierbahn und einer gewellten Papierbahn hergestellt. Einwellige Wellpappe wird beispielsweise aus zwei flachen Papierbahnen und einer gewellten Papierbahn hergestellt. Darüber hinaus lassen sich noch zweiwellige, dreiwellige oder mehrwellige Wellpappe unterscheiden, die im Wesentlichen durch ihre Anzahl an gewellten Papierbahnen definiert werden. Diese können dabei jeweils wieder einseitig oder einwellig ausgeführt sein. Beispielsweise wird eine zweiwellige Wellpappe aus drei flachen Papierbahnen und zwei gewellten Papierbahnen, die sich je zwischen zwei flachen Papierbahnen befinden, hergestellt. Der Begriff Wellpappe kann im Rahmen dieser Anmeldung sowohl als das Endlosprodukt bestehend aus mindestens einer flachen Papierbahn und einer gewellten Papierbahn, also eine Wellpappenbahn, als auch als Wellpappenprodukt, also nicht mehr in Endlosform, verstanden werden. Der Begriff Produktbahn bezeichnet ihm Rahmen dieser Anmeldung generell ein Erzeugnis aus zumindest zwei Bahnen beziehungsweise zwei Papierbahnen.

Da zumindest die gewellte Papierbahn unmittelbar vor dem Klebeauftrag und dem Inkontaktbringen vor allem durch vorgelagerte Prozessschritte in aufgeheiztem Zustand ist, erhitzt sich ebenfalls das Klebemittel beim Klebeauftrag auf die jeweilige Papierbahn beziehungsweise spätestens beim Inkontaktbringen der beiden zu verklebenden Papierbahnen. Das heißt im Bereich der Verklebung, also im Kontaktbereich der zu verklebenden Papierbahnen, liegt eine andere Temperaturcharakteristik vor als in nicht verklebten beziehungsweise in nicht kontaktierten Bereichen der Wellpappe. Insbesondere die Temperaturentwicklung zu unterschiedlichen Prozesspunkten verläuft an den eben beschriebenen Bereichen sehr unterschiedlich. Kontaktierte Bereiche mit Klebemittelauftrag kühlen sich dabei beispielsweise langsamer ab, als Bereiche die weder mit Klebemittel beauftragt sind noch kontaktiert sind. Somit lässt sich anhand der Temperatur der Wellpappe, beziehungsweise anhand der abgestrahlten Wärme von einer Wellpappenoberfläche die Qualität der Wellpappenverklebung zuverlässig beurteilen. Mit Wellpappenoberfläche kann beispielsweise im Falle einer einseitigen Wellpappe, sowohl die Oberfläche der Deckbahn als auch die Oberfläche der gewellten Papierbahn gemeint sein.

Mit anderen Worten ausgedrückt bedeutet dies, dass das Klebemittel während des Klebevorgangs in der Klebestation durch den Kontakt mit insbesondere der gewellten Papierbahn erhitzt wird und unmittelbar danach zusammen mit der Wellpappe abkühlt. Je nachdem wieviel Klebemittel an einer Klebestelle zwischen den zu verklebenden Papierbahnen aufgetragen wurde, kühlt sich das Klebemittel und damit auch die jeweilige Papierbahn bzw. Papierbahnoberfläche im Bereich der Klebestelle schneller oder langsamer ab. Ebenso kühlt sich das Klebemittel und auch die jeweilige Papierbahn bzw. Papierbahnoberfläche langsamer oder schneller ab, je nachdem ob die zu verklebenden Papierbahnen nach dem Durchlaufen der Klebestation in vollem Umfang in Kontakt bleiben oder sich teilweise oder ganz lösen. Wurde beispielsweise zu wenig Klebemittel aufgetragen oder haben sich die zu verklebenden Papierbahnen nach dem Inkontaktbringen wieder voneinander gelöst, so wird eine geringere Temperatur detektiert als bei einer fehlerfreien Verklebung. Umgekehrt führt ein zu großer Klebemittelauftrag zu einer Detektion einer höheren Temperatur als bei einer fehlerfreien Verklebung. Somit ermöglicht das erfindungsgemäße Messsystem eine zuverlässige Beurteilung der real vorhandenen Verklebung, also dem Zustand der Verklebung nach dem Klebevorgang. Weiterhin kann die Überprüfung zerstörungsfrei und ohne Beeinflussung des Betriebsablaufs durchgeführt werden.

In Ausgestaltungen, die mit der vorangehenden Ausgestaltung kombinierbar sind, kann die Messeinrichtung vorzugsweise eine bildgebende Messeinrichtung umfassen. Dies ermöglicht beispielsweise die Etablierung einer thermografischen Auswertung der Messung.

In Ausgestaltungen, die mit irgendeiner der vorangehenden Ausgestaltungen kombinierbar sind, kann die Messeinrichtung ausgelegt sein, eine Temperaturverteilung thermografisch darzustellen und zu analysieren. Eine thermografische Darstellung der Temperaturverteilung kann einerseits einem Bediener der Messeinrichtung die Erfassung des Messergebnisses erleichtern. Beispielsweise kann ein Bediener durch die thermografische Darstellung zügig eine qualitative Einschätzung der Sachlage abgeben. Andererseits kann durch eine thermografische Darstellung die Möglichkeit einer automatisierten grafischen Auswertung der Messung geschaffen werden.

In Ausgestaltungen, die mit irgendeiner der vorangehenden Ausgestaltungen kombinierbar sind, kann die Messeinrichtung ausgelegt sein, die Temperatur, vorzugsweise die Oberflächentemperatur der Wellpappe zu erfassen. Weiterhin kann die Messeinrichtung ausgelegt sein, eine Temperaturverteilung der Wellpappe zu beurteilen. Alternativ oder zusätzlich kann die Messeinrichtung ausgelegt sein, eine Temperaturverteilung der Wellpappe, vorzugsweise eine Temperaturverteilung der Wellpappe entlang der Bahnbreite zu erfassen. Da die Temperatur bei einer fehlerfreien Verklebung normalerweise homogen bzw. entsprechend eines bestimmten Musters, insbesondere über die Bahnbreite verteilt ist, kann anhand der Temperaturverteilung der Wellpappe eine ganzheitliche Erfassung der Qualität der Wellpappenverklebung erfolgen. Das heißt die Messeinrichtung kann ausgelegt sein, die Verklebegüte einer Wellpappenverklebung, vorzugsweise anhand einer Temperaturverteilung der Wellpappe zu beurteilen.

In Ausgestaltungen, die mit irgendeiner der vorangehenden Ausgestaltungen kombinierbar sind, kann die Messeinrichtung ausgelegt sein, eine Temperaturverteilung der Wellpappe zeilenförmig und/oder matrixförmig zu beurteilen. Alternativ oder zusätzlich kann die Messeinrichtung eine Infrarot-Kamera umfassen.

In Ausgestaltungen, die mit irgendeiner der vorangehenden Ausgestaltungen kombinierbar sind, kann die Messeinrichtung weiterhin eine Auswerteeinrichtung zur Auswertung der erfassten elektromagnetischen Wellen im Infrarot-Bereich umfassen. Die Auswerteeinrichtung kann dabei einen Computer mit einer Speichereinrichtung umfassen. Auf der Speichereinrichtung kann ein Computerprogramm zur Überprüfung des Herstellungsprozesses von Verklebungen gespeichert sein. Dabei können insbesondere Verklebungen einer ersten Papierbahn mit mindestens einer zweiten Papierbahn zu einer Produktbahn überprüft werden. Das Computerprogramm kann dabei ausgelegt sein die folgenden Schritte auszuführen:
- Detektieren thermischer Strahlung der Produktbahn nach dem Verkleben der ersten Papierbahn mit der mindestens zweiten Papierbahn,
- Erstellen thermografischer Daten basierend auf der thermischen Strahlung,
- Auswerten der thermografischen Daten, und
- Beurteilen der Verklebegüte der Papierverklebung anhand der Auswertung.
Durch diese besonders vorteilhaften Ausgestaltungen kann eine automatisierte Qualitätsüberprüfung bereitgestellt werden. Weiterhin kann durch die automatisierte Überprüfung eine 100% Kontrolle der Verklebungen sichergestellt werden. Durch die automatisierte und frühe Erkennung eventueller Fehler kurz nach der Klebestation lässt sich der Herstellungsprozess bzw. der Verklebeprozess zügig anpassen, wodurch der Ausschuss fehlerhafter Erzeugnisse massiv reduziert werden kann im Vergleich zu einer Qualitätskontrolle zu einem späteren Zeitpunkt im Produktionsprozess, beispielsweise im Warenausgang.

In Ausgestaltungen, die mit der vorangehenden Ausgestaltung kombinierbar sind, kann das Detektieren ein Detektieren elektromagnetischer Strahlung im Infrarot-Bereich umfassen. Alternativ oder zusätzlich kann die thermische Strahlung der Produktbahn in einem Erfassungsbereich, der sich zeilenförmig in y-Richtung der Produktbahn und/oder zweidimensional in x- und y-Richtung der Produktbahn erstreckt, detektiert werden. Alternativ oder zusätzlich können die thermografischen Daten in einem Auswertebereich, der vorzugsweise ein zweidimensionaler Datenbereich in x- und y-Richtung ist, erstellt werden. Alternativ oder zusätzlich kann die Beurteilung der Verklebegüte anhand einer geometrischen Auswertung der thermografischen Daten erfolgen. Durch derartige vorteilhafte Ausgestaltungen kann nicht nur überprüft werden, ob der Klebemittelauftrag ausreichend oder zu umfangreich war, sondern es kann auch unabhängig vom Klebemittelauftrag überprüft werden, ob eine erfolgreiche Verklebung zustande gekommen ist. Das heißt es kann überprüft werden, ob die mindestens zwei zu verklebenden Papierbahnen nach dem Klebemittelauftrag und nach dem Inkontaktbringen auch in Kontakt geblieben sind. Dies ist deshalb möglich, da die zwei zu verklebenden Papierbahnen bzw. auch deren Oberflächen eine bestimmte Temperaturverteilung und dementsprechend auch spezifisches thermografische Eigenschaften auf ihren Oberflächen aufweisen, anhand derer Rückschlüsse auf den Verklebezustand gezogen werden können.

In Ausgestaltungen, die mit irgendeiner der zwei vorangehenden Ausgestaltungen kombinierbar sind, können Fehlstellen der Verklebung durch geometrische Inhomogenitäten in den thermografischen Daten detektiert werden.

In Ausgestaltungen, die mit der vorangehenden Ausgestaltung kombinierbar sind, können Fehlstellen anhand geometrischer Inhomogenitäten, die von einem vorgegebenen Muster in den thermografischen Daten abweichen, identifiziert werden. Zu diesen geometrischen Inhomogenitäten gehören insbesondere kreisförmige, rechteckige und/oder im Wesentlichen über die gesamte Bahnbreite in y-Richtung verlaufende geometrische Inhomogenitäten.

In Ausgestaltungen, die mit irgendeiner der vier vorangehenden Ausgestaltungen kombinierbar sind, kann die Auswertung eine ortsaufgelöste Bestimmung von geometrischen Dimensionen einer oder mehrerer isothermer Bereiche und/oder Teilbereiche in den thermografischen Daten umfassen. Die geometrischen Dimensionen des einen oder der mehreren isothermen Bereiche und/oder Teilbereiche kann dabei bevorzugt die Länge in x-Richtung und/oder die Breite in y-Richtung umfassen.

In Ausgestaltungen, die mit der vorangehenden Ausgestaltung kombinierbar sind, kann die Verklebegüte anhand eines Vergleichs der geometrischen Dimensionen des einen oder der mehreren isothermen Bereiche und/oder Teilbereiche einer Verklebung mit Referenzwerten der geometrischen Dimensionen beurteilt werden.

In Ausgestaltungen, die mit irgendeiner der sechs vorangehenden Ausgestaltungen kombinierbar sind, kann die Auswertung die Bildung eines Integrals über einen zeilenförmigen Teilbereich der thermografischen Daten einer oder mehrerer isothermer Bereiche und/oder Teilbereiche in x-Richtung umfassen. Dabei kann die Verklebegüte anhand eines Vergleichs des Integrals des einen oder der mehreren isothermen Bereiche und/oder Teilbereiche einer Verklebung mit Referenzwerten des jeweiligen Integrals beurteilt werden.

In Ausgestaltungen, die mit irgendeiner der sieben vorangehenden Ausgestaltungen kombinierbar sind, kann die Auswertung die Bildung eines Flächenintegrals über einen Teilbereich der thermografischen Daten einer oder mehrerer isothermer Bereiche und/oder Teilbereiche in x-Richtung und y-Richtung umfassen. Hierbei kann die Verklebegüte anhand eines Vergleichs des Flächenintegrals des einen oder der mehreren isothermen Bereiche und/oder Teilbereiche einer Verklebung mit Referenzwerten des jeweiligen Flächenintegrals beurteilt werden.

In Ausgestaltungen, die mit irgendeiner der drei vorangehenden Ausgestaltungen kombinierbar sind, können die Referenzwerte vorbestimmte Referenzwerte umfassen. Alternativ oder zusätzlich können die Referenzwerte über einen Durchschnitt der vorherig überprüften Verklebungen gebildet werden. Insbesondere können die Referenzwerte dabei über einen Durchschnitt der zwei vorherig überprüften Verklebungen, bevorzugt der fünf vorherig überprüften Verklebungen und besonders bevorzugt der mindestens zehn vorherig überprüften Verklebungen gebildet werden.

In Ausgestaltungen, die mit irgendeiner der vier vorangehenden Ausgestaltungen kombinierbar sind, kann die Verklebegüte als ausreichend beurteilt werden, wenn der jeweilige Vergleich eine maximale Abweichung von 0% bis 50%, bevorzugt von 0% bis 20% und besonders bevorzugt von 0% bis 5% ergibt.

In Ausgestaltungen, die mit irgendeiner der neun vorangehenden Ausgestaltungen kombinierbar sind, kann das Detektieren der thermischen Strahlung mit einer Infrarot-Kamera durchgeführt werden.

In Ausgestaltungen, die mit irgendeiner der zehn vorangehenden Ausgestaltungen kombinierbar sind, kann das Computerprogramm ausgelegt sein, in-line beim Herstellungsprozess von Papierverklebungen, vorzugsweise Wellpappenverklebungen ausgeführt zu werden.

Die Erfindung umfasst weiterhin eine Wellpappenmaschine. Die Wellpappenmaschine umfasst eine Transporteinrichtung zum Transport von Wellpappe in einer Transportrichtung. Weiterhin umfasst die Wellpappenmaschine eine Klebestation, die zum Verkleben einer ersten Papierbahn mit mindestens einer zweiten Papierbahn ausgelegt ist. Weiterhin umfasst die Wellpappenmaschine ein Messsystem zur Überprüfung des Herstellungsprozesses von Verklebungen der Wellpappe. Das Messsystem kann dabei ein Messsystem nach irgendeiner der vorhergehen Ausgestaltungen sein.

In Ausgestaltungen der erfindungsgemäßen Wellpappenmaschine, die mit der vorangehenden Ausgestaltung kombinierbar sind, kann die Wellpappenmaschine weiterhin Heizelemente umfassen, die an der Transporteinrichtung angeordnet sind.

In Ausgestaltungen der erfindungsgemäßen Wellpappenmaschine, die mit irgendeiner der zwei vorangehenden Ausgestaltung kombinierbar sind, kann die Wellpappenmaschine weiterhin eine Ausschusseinrichtung umfassen. Die Auschusseinrichtung kann dabei in Transportrichtung hinter dem Messsystem angeordnet sein. Weiterhin kann die Ausschusseinrichtung ausgelegt sein, Wellpappe mit einer Verklebegüte unterhalb eines Grenzwerts auszusortieren.

In Ausgestaltungen der erfindungsgemäßen Wellpappenmaschine, die mit irgendeiner der drei vorangehenden Ausgestaltung kombinierbar sind, kann das Messsystem auf eine Oberflächenseite der ersten Papierbahn gerichtet sein, deren Normalenvektor in die entgegengesetzte Richtung der zweiten Papierbahn gerichtet ist. Alternativ oder zusätzlich kann das Messsystem auf eine Oberflächenseite der mindestens zweiten Papierbahn gerichtet sein, deren Normalenvektor in die entgegengesetzte Richtung der ersten Papierbahn gerichtet ist. Das bedeutet, dass die Messeinrichtung lediglich auf eine Oberfläche einer Papierbahn gerichtet sein muss und somit auch nur auf einer Oberflächenseite der Papierbahn angeordnet sein braucht. Alternativ kann die Messeinrichtung aber auch zwei Einheiten umfassen, die auf beiden Seiten der Papierbahn angeordnet sind, um beide Oberflächen der Papierbahn zu erfassen.

Die Erfindung umfasst weiterhin ein Verfahren zur Überprüfung des Herstellungsprozesses von Verklebungen einer ersten Papierbahn mit mindestens einer zweiten Papierbahn zu einer Produktbahn, unter Verwendung eines Messsystems. Das Verfahren umfasst dabei die folgenden Schritte:
- Detektieren thermischer Strahlung der Produktbahn nach dem Verkleben der ersten Papierbahn mit der mindestens zweiten Papierbahn,
- Erstellen thermografischer Daten basierend auf der thermischen Strahlung,
- Auswerten der thermografischen Daten, und
- Beurteilen der Verklebegüte der Papierverklebung anhand der Auswertung.
Durch diese besonders vorteilhaften Ausgestaltungen kann eine automatisierte Qualitätsüberprüfung bereitgestellt werden. Weiterhin kann durch die automatisierte Überprüfung eine 100% Kontrolle der Verklebungen sichergestellt werden. Durch die automatisierte und frühe Erkennung eventueller Fehler kurz nach der Klebestation lässt sich der Herstellungsprozess bzw. der Verklebeprozess zügig anpassen, wodurch der Ausschuss fehlerhafter Erzeugnisse massiv reduziert werden kann im Vergleich zu einer Qualitätskontrolle zu einem späteren Zeitpunkt im Produktionsprozess, beispielsweise im Warenausgang.

In Ausgestaltungen des erfindungsgemäßen Verfahrens, die mit der vorangehenden Ausgestaltung kombinierbar sind, kann das Detektieren ein Detektieren elektromagnetischer Strahlung im Infrarot-Bereich umfassen.

In Ausgestaltungen des erfindungsgemäßen Verfahrens, die mit irgendeiner der zwei vorangehenden Ausgestaltung kombinierbar sind, kann die thermische Strahlung der Produktbahn in einem Erfassungsbereich detektiert werden. Der Erfassungsbereich kann sich dabei zeilenförmig in y-Richtung der Produktbahn erstrecken. Alternativ kann sich der Erfassungsbereich dabei zweidimensional in x- und y-Richtung der Produktbahn erstrecken.

In Ausgestaltungen des erfindungsgemäßen Verfahrens, die mit irgendeiner der drei vorangehenden Ausgestaltungen kombinierbar sind, können die thermografischen Daten in einem Auswertebereich erstellt werden. Der Auswertebereich kann dabei insbesondere ein zweidimensionaler Datenbereich in x- und y-Richtung sein.

In Ausgestaltungen des erfindungsgemäßen Verfahrens, die mit irgendeiner der vier vorangehenden Ausgestaltung kombinierbar sind, kann die Beurteilung der Verklebegüte anhand einer geometrischen Auswertung der thermografischen Daten erfolgen. Durch derartige vorteilhafte Ausgestaltungen kann nicht nur überprüft werden, ob der Klebemittelauftrag ausreichend oder zu umfangreich war, sondern es kann auch unabhängig vom Klebemittelauftrag überprüft werden, ob eine erfolgreiche Verklebung zustande gekommen ist. Das heißt es kann überprüft werden, ob die zwei zu verklebenden Papierbahnen nach dem Klebemittelauftrag und nach dem Inkontaktbringen auch in Kontakt geblieben sind. Dies ist deshalb möglich, da die zwei zu verklebenden Papierbahnen bzw. deren Oberflächen eine bestimmte Temperaturverteilung und dementsprechend auch spezifisches thermografische Eigenschaften auf ihren Oberflächen aufweisen, anhand derer Rückschlüsse auf den Verklebezustand gezogen werden können.

In Ausgestaltungen des erfindungsgemäßen Verfahrens, die mit irgendeiner der fünf vorangehenden Ausgestaltung kombinierbar sind, können Fehlstellen der Verklebung durch geometrische Inhomogenitäten in den thermografischen Daten detektiert werden.

In Ausgestaltungen des erfindungsgemäßen Verfahrens, die mit der vorangehenden Ausgestaltung kombinierbar sind, können Fehlstellen anhand geometrischer Inhomogenitäten, die von einem vorgegebenen Muster in den thermografischen Daten abweichen, identifiziert werden. Zu diesen geometrischen Inhomogenitäten insbesondere kreisförmige, rechteckige und/oder im Wesentlichen über die gesamte Bahnbreite in y-Richtung verlaufende geometrische Inhomogenitäten.

In Ausgestaltungen des erfindungsgemäßen Verfahrens, die mit irgendeiner der sechs vorangehenden Ausgestaltung kombinierbar sind, kann die Auswertung eine ortsaufgelöste Bestimmung von geometrischen Dimensionen einer oder mehrerer isothermer Bereiche und/oder Teilbereiche in den thermografischen Daten umfassen. Die geometrischen Dimensionen des einen oder der mehreren isothermen Bereiche und/oder Teilbereiche kann dabei bevorzugt die Länge in x-Richtung und/oder die Breite in y-Richtung umfassen.

In Ausgestaltungen des erfindungsgemäßen Verfahrens, die mit der vorangehenden Ausgestaltung kombinierbar sind, kann die Verklebegüte anhand eines Vergleichs der geometrischen Dimensionen des einen oder der mehreren isothermen Bereiche und/oder Teilbereiche einer Verklebung mit Referenzwerten der geometrischen Dimensionen beurteilt werden.

In Ausgestaltungen des erfindungsgemäßen Verfahrens, die mit irgendeiner der acht vorangehenden Ausgestaltungen kombinierbar sind, kann die Auswertung die Bildung eines Integrals über einen zeilenförmigen Teilbereich der thermografischen Daten einer oder mehrerer isothermer Bereiche und/oder Teilbereiche in x-Richtung umfassen. Dabei kann die Verklebegüte zusätzlich anhand eines Vergleichs des Integrals des einen oder der mehreren isothermen Bereiche und/oder Teilbereiche einer Verklebung mit Referenzwerten des jeweiligen Integrals beurteilt werden.

In Ausgestaltungen des erfindungsgemäßen Verfahrens, die mit irgendeiner der neun vorangehenden Ausgestaltungen kombinierbar sind, kann die Auswertung die Bildung eines Flächenintegrals über einen Teilbereich der thermografischen Daten einer oder mehrerer isothermer Bereiche und/oder Teilbereiche in x-Richtung und y-Richtung umfassen. Hierbei kann die Verklebegüte zusätzlich anhand eines Vergleichs des Flächenintegrals des einen oder der mehreren isothermen Bereiche und/oder Teilbereiche einer Verklebung mit Referenzwerten des jeweiligen Flächenintegrals beurteilt wird.

In Ausgestaltungen des erfindungsgemäßen Verfahrens, die mit irgendeiner der drei vorangehenden Ausgestaltungen kombinierbar sind, können die Referenzwerte vorbestimmte Referenzwerte umfassen. Alternativ oder zusätzlich können die Referenzwerte über einen Durchschnitt der vorherig überprüften Verklebungen gebildet werden. Insbesondere können die Referenzwerte dabei über einen Durchschnitt der zwei vorherig überprüften Verklebungen, bevorzugt der fünf vorherig überprüften Verklebungen und besonders bevorzugt der mindestens zehn vorherig überprüften Verklebungen gebildet werden.

In Ausgestaltungen des erfindungsgemäßen Verfahrens, die mit irgendeiner der vier vorangehenden Ausgestaltungen kombinierbar sind, kann die Verklebegüte als ausreichend beurteilt werden, wenn der jeweilige Vergleich eine maximale Abweichung von 0% bis 50%, bevorzugt von 0% bis 20% und besonders bevorzugt von 0% bis 5% ergibt.

In Ausgestaltungen des erfindungsgemäßen Verfahrens, die mit irgendeiner der elf vorangehenden Ausgestaltungen kombinierbar sind, kann das Detektieren der thermischen Strahlung mit einer Infrarot-Kamera durchgeführt werden.

In Ausgestaltungen des erfindungsgemäßen Verfahrens, die mit irgendeiner der zwölf vorangehenden Ausgestaltungen kombinierbar sind, kann das Computerprogramm ausgelegt sein, in-line beim Herstellungsprozess von Papierverklebungen, vorzugsweise Wellpappenverklebungen ausgeführt zu werden.

In Ausgestaltungen des erfindungsgemäßen Verfahrens, die mit irgendeiner der dreizehn vorangehenden Ausgestaltungen kombinierbar sind, kann die thermische Strahlung von einer Oberflächenseite der ersten Papierbahn detektiert werden, deren Normalenvektor in die entgegengesetzte Richtung der zweiten Papierbahn gerichtet ist. Alternativ oder zusätzlich kann die thermische Strahlung von einer mindestens zweiten Papierbahn detektiert werden, deren Normalenvektor in die entgegengesetzte Richtung der ersten Papierbahn gerichtet ist. In Ausgestaltungen des erfindungsgemäßen Verfahrens, die mit irgendeiner der vierzehn vorangehenden Ausgestaltungen kombinierbar sind, kann die Papierverklebung insbesondere eine Wellpappenverklebung sein.

In Ausgestaltungen des erfindungsgemäßen Verfahrens, die mit irgendeiner der fünfzehn vorangehenden Ausgestaltungen kombinierbar sind, kann die Produktbahn insbesondere eine Wellpappenbahn sein.

In Ausgestaltungen des erfindungsgemäßen Verfahrens, die mit irgendeiner der sechzehn vorangehenden Ausgestaltungen kombinierbar sind, kann das Verfahren mit einem Messsystem gemäß irgendeiner der Ausgestaltungen 1 bis 15 durchgeführt wird.

### Kurzbeschreibung der Figuren

- **Figur 1**: zeigt eine Seitenansicht des erfindungsgemäßen Messsystems mit relativer Positionierung in einer Wellpappenanlage
- **Figur 2a-c**: zeigen eine isometrische Ansicht der Materialbahn, eine dazugehörige thermografische Ansicht und ein Temperaturverteilungsdiagramm einer fehlerfreien Verklebung
- **Figur 3a-c**: zeigen eine isometrische Ansicht der Materialbahn, eine dazugehörige thermografische Ansicht und ein Temperaturverteilungsdiagramm einer fehlerbehafteten Verklebung mit geometrischer Auswertung
- **Figur 4**: zeigt ein Temperaturverteilungsdiagramm mit thermografischen Daten einer fehlerfreien Materialbahn wie aus den Fig. 2a-c mit integraler Auswertung
- **Figur 5**: zeigt ein Temperaturverteilungsdiagramm mit thermografischen Daten einer fehlerbehafteten Materialbahn wie aus den Fig. 3a-c mit integraler Auswertung
- **Figur 6**: zeigt eine isometrische Ansicht der erfindungsgemäßen Wellpappenmaschine mit Messsystem und Klebestation

### Detaillierte Beschreibung

Im Folgenden werden anhand der Figuren Ausführungsbeispiele für das erfindungsgemäße Messsystem 1 beschrieben. Im Rahmen dieser Anmeldung bezieht sich der Begriff Bahnbreite 92a auf eine Ausdehnung einer Transporteinrichtung bzw. einer Produktbahn/Wellpappe in y-Richtung 92 und der Begriff Transportrichtung 94a auf eine Förderrichtung der Transporteinrichtung bzw. der Produktbahn/Wellpappe in x-Richtung 94. Mit dem Begriff Papierbahn ist hierbei ein Endlosmaterial aus Papier zu verstehen. Dies kann beispielsweise eine gewellte Papierbahn oder eine flache Papierbahn, auch Deckbahn genannt, sein. Einseitige Wellpappe wird beispielsweise aus einer flachen Papierbahn und einer gewellten Papierbahn hergestellt. Einwellige Wellpappe wird beispielsweise aus zwei flachen Papierbahnen und einer gewellten Papierbahn hergestellt. Darüber hinaus lassen sich noch zweiwellige, dreiwellige oder mehrwellige Wellpappe unterscheiden, die im Wesentlichen durch ihre Anzahl an gewellten Papierbahnen definiert werden. Diese können dabei jeweils wieder einseitig oder einwellig ausgeführt sein. Beispielsweise wird eine zweiwellige Wellpappe aus drei flachen Papierbahnen und zwei gewellten Papierbahnen, die sich je zwischen zwei flachen Papierbahnen befinden, hergestellt. Der Begriff Wellpappe kann im Rahmen dieser Anmeldung sowohl als das Endlosprodukt bestehend aus mindestens einer flachen Papierbahn und einer gewellten Papierbahn, also eine Wellpappenbahn, als auch als Wellpappenprodukt, also nicht mehr in Endlosform, verstanden werden. Der Begriff Produktbahn bezeichnet ihm Rahmen dieser Anmeldung generell ein Erzeugnis aus zumindest zwei Bahnen beziehungsweise zwei Papierbahnen.

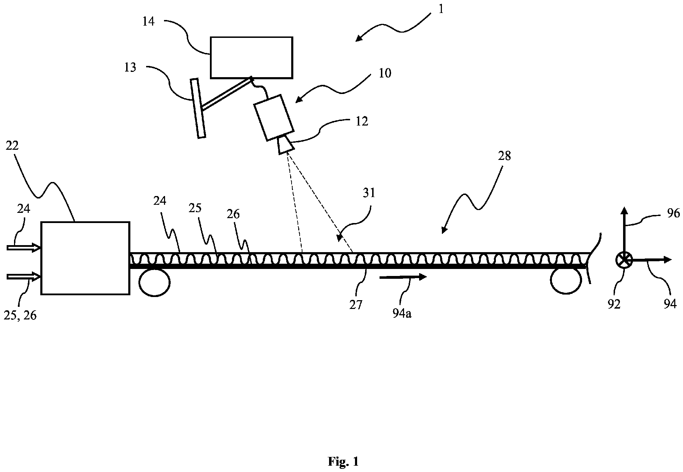

In **Fig. 1** ist ein beispielhaftes Messsystem 1 zur Verwendung in einer Wellpappenmaschine dargestellt. Das erfindungsgemäße Messsystem 1 zur Verwendung in einer Wellpappenmaschine umfasst in einer ersten Variante eine Messeinrichtung 10. Die Messeinrichtung 10 kann dabei elektromagnetische Wellen im Infrarot-Bereich erfassen und ist auf einen Bereich 31 einer Transporteinrichtung 27 der Wellpappenmaschine in Transportrichtung 94a hinter einer Klebestation 22 der Wellpappenmaschine gerichtet.

Dadurch, dass die Messeinrichtung 10 Infrarotstrahlung erfassen kann, ergibt sich grundsätzlich die Möglichkeit, dass eine Temperatur der Wellpappe 28 erfasst werden kann. Ausgehend von der Temperatur können Rückschlüsse auf die Qualität der Wellpappe 28 geschlossen werden. Dies ist gerade deshalb möglich, weil die Messstation 1 auf einen Bereich der Transporteinrichtung 27 in Transportrichtung 94a hinter der Klebestation 22 gerichtet ist. Mit anderen Worten bedeutet dies, dass die Wellpappe 28 nach dem Verkleben, also nach dem Klebemittelauftrag und dem Inkontaktbringen mindestens zweier zu verklebender Papierbahnen (24, 25, 26) überprüft werden kann. Das heißt die Wellpappenverklebung wird erst dann überprüft, wenn der Klebevorgang nahezu abgeschlossen ist und sich das Klebemittel im Aushärtevorgang befindet. Der Gelierpunkt des Klebemittels kann dabei in einem Temperaturbereich von 40°C bis 70°C und bevorzugt in einem Temperaturbereich von 50°C bis 60°C liegen. Besonders bevorzugt kann der Gelierpunkt des Klebemittels bei ca. 55°C liegen.

Da zumindest die gewellte Papierbahn 25 unmittelbar vor dem Klebeauftrag und dem Inkontaktbringen vor allem durch vorgelagerte Prozessschritte in aufgeheiztem Zustand ist, erhitzt sich ebenfalls das Klebemittel beim Klebeauftrag auf die jeweilige Papierbahn (24, 25, 26) beziehungsweise spätestens beim Inkontaktbringen der beiden zu verklebenden Papierbahnen (24, 25, 26). Das heißt im Bereich der Verklebung, also im Kontaktbereich der zu verklebenden Papierbahnen (24, 25, 26), liegt eine andere Temperaturcharakteristik vor als in nicht verklebten beziehungsweise in nicht kontaktierten Bereichen der Wellpappe 28. Insbesondere die Temperaturentwicklung zu unterschiedlichen Prozesspunkten verläuft an den eben beschriebenen Bereichen sehr unterschiedlich. Kontaktierte Bereiche mit Klebemittelauftrag kühlen sich dabei beispielsweise langsamer ab, als Bereiche die weder mit Klebemittel beauftragt sind noch kontaktiert sind. Die Temperaturen an der Oberfläche der Wellpappe beziehungsweise an den mindestens zwei Papierbahnen kann dabei je nach Prozessort zwischen 30°C und 130°C, insbesondere zwischen 60°C und 120°C liegen. Somit lässt sich anhand der Temperatur der Wellpappe 28, beziehungsweise anhand der abgestrahlten Wärme von einer Wellpappenoberfläche die Qualität der Wellpappenverklebung zuverlässig beurteilen. Mit Wellpappenoberfläche kann beispielsweise im Falle einer einseitigen Wellpappe, sowohl die Oberfläche der Deckbahn (24, 26) als auch die Oberfläche der gewellten Papierbahn (25) gemeint sein.

Mit anderen Worten ausgedrückt bedeutet dies, dass das Klebemittel während des Klebevorgangs in der Klebestation 22 durch den Kontakt mit insbesondere der gewellten Papierbahn 25 erhitzt wird und unmittelbar danach zusammen mit der Wellpappe 28 abkühlt. Je nachdem wieviel Klebemittel an einer Klebestelle zwischen den zu verklebenden Papierbahnen (24, 25, 26) aufgetragen wurde, kühlt sich das Klebemittel und damit auch die jeweilige Papierbahn (24, 25, 26) bzw. Papierbahnoberfläche im Bereich der Klebestelle schneller oder langsamer ab. Ebenso kühlt sich das Klebemittel und auch die jeweilige Papierbahn (24, 25, 26) bzw. Papierbahnoberfläche langsamer oder schneller ab, je nachdem ob die zu verklebenden Papierbahnen nach dem Durchlaufen der Klebestation 22 in vollem Umfang in Kontakt bleiben oder sich teilweise oder ganz lösen. Wurde beispielsweise zu wenig Klebemittel aufgetragen oder haben sich die zu verklebenden Papierbahnen (24, 25, 26) nach dem Inkontaktbringen wieder voneinander gelöst, so wird eine geringere Temperatur detektiert als bei einer fehlerfreien Verklebung. Umgekehrt führt ein zu großer Klebemittelauftrag zu einer Detektion einer höheren Temperatur als bei einer fehlerfreien Verklebung. Somit ermöglicht das erfindungsgemäße Messsystem 1 eine zuverlässige Beurteilung der real vorhandenen Verklebung, also dem Zustand der Verklebung nach dem Klebevorgang. Weiterhin kann die Überprüfung zerstörungsfrei und ohne Beeinflussung des Betriebsablaufs durchgeführt werden.

Weiterhin zeigt **Fig. 1** ein Koordinatensystem (92, 94, 96). Als x-Richtung 94 wird hierbei eine Richtung entlang der Beförderung der Wellpappe verstanden. Die y-Richtung 92 bezeichnet eine Richtung, welche orthogonal zur x-Richtung 94 innerhalb der Bahnebene liegt. Die z-Richtung ist als eine Richtung zu verstehen, welche sich in Normalenrichtung von der Bahnebene, also orthogonal zur x-Richtung 94 und orthogonal zur y-Richtung 92 erstreckt.

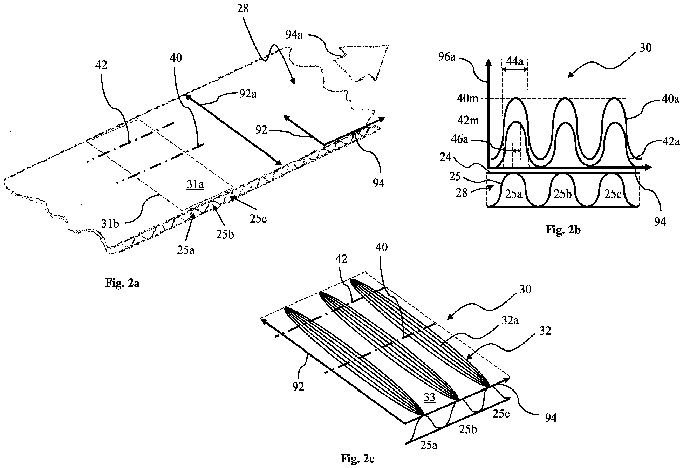

Die **Fig. 2a** zeigt einen Ausschnitt der Transporteinrichtung 27 beziehungsweise. der Wellpappenbahn 28 mit einem Erfassungsbereich (31, 31a, 31b) des Messsystems 1 beziehungsweise der Messeinrichtung 10. Wie hierbei insbesondere zu erkennen ist, kann die Messeinrichtung 10 ausgelegt sein, eine Temperaturverteilung der Wellpappe 28 zeilenförmig 31b, beispielsweise entlang der gestrichelten Erfassungslinie 31b, die in y-Richtung 92 verläuft zu beurteilen. Alternativ oder zusätzlich kann die Messeinrichtung 10 ausgelegt sein, eine Temperaturverteilung der Wellpappe 28 matrixförmig 31a, beispielsweise anhand des Erfassungsbereich 31a, der sich in x-Richtung 94 und in y-Richtung 92 erstreckt, zu beurteilen. Alternativ oder zusätzlich kann die Messeinrichtung 10 eine Infrarot-Kamera 12 umfassen (siehe **Fig. 1**).

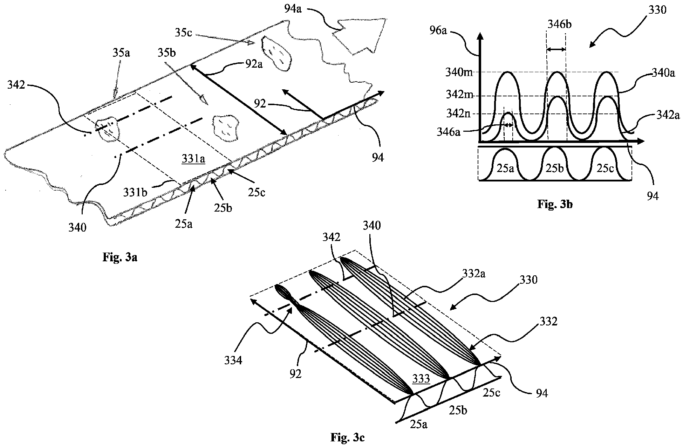

Weiterhin kann die Messeinrichtung 10 vorzugsweise eine bildgebende Messeinrichtung 13 umfassen (siehe **Fig. 1**). Dies ermöglicht beispielsweise die Etablierung einer thermografischen Auswertung der Messung. Weiterhin kann die Messeinrichtung 10 ausgelegt sein, eine Temperaturverteilung (40a, 42a, 340a, 342a) thermografisch darzustellen und zu analysieren (siehe insbesondere **Fig. 2b****-c** und **Fig. 3b****-c**). Eine thermografische Darstellung der Temperaturverteilung (40a, 42a, 340a, 342a) kann einerseits einem Bediener der Messeinrichtung 10 die Erfassung des Messergebnisses erleichtern. Beispielsweise kann ein Bediener durch die thermografische Darstellung zügig eine qualitative Einschätzung der Sachlage abgeben. Andererseits kann durch eine thermografische Darstellung die Möglichkeit einer automatisierten grafischen Auswertung der Messung geschaffen werden.

In Ausgestaltungen, die mit irgendeiner der vorangehenden Ausgestaltungen kombinierbar sind, kann die Messeinrichtung 10 ausgelegt sein, die Temperatur, vorzugsweise die Oberflächentemperatur der Wellpappe 28 zu erfassen. Weiterhin kann die Messeinrichtung 10 ausgelegt sein, eine Temperaturverteilung (40a, 42a, 340a, 342a) der Wellpappe 28 zu beurteilen. Alternativ oder zusätzlich kann Messeinrichtung 10 ausgelegt sein, eine Temperaturverteilung (40a, 42a, 340a, 342a) der Wellpappe 28, vorzugsweise eine Temperaturverteilung der Wellpappe entlang der Bahnbreite 92a zu erfassen. Da die Temperatur bei einer fehlerfreien Verklebung normalerweise homogen bzw. entsprechend eines bestimmten Musters 32 (siehe **Fig. 2c**, wird später im Detail erläutert), insbesondere über die Bahnbreite 92a verteilt ist, kann anhand der Temperaturverteilung (40a, 42a, 340a, 342a) der Wellpappe 28 eine ganzheitliche Erfassung der Qualität der Wellpappenverklebung erfolgen. Das heißt die Messeinrichtung 10 kann ausgelegt sein, die Verklebegüte einer Wellpappenverklebung, vorzugsweise anhand einer Temperaturverteilung (40a, 42a, 340a, 342a) der Wellpappe 28 zu beurteilen.

Wie in der **Fig. 1** zu erkennen ist, kann die Messeinrichtung 10 weiterhin eine Auswerteeinrichtung 14 zur Auswertung der erfassten elektromagnetischen Wellen im Infrarot-Bereich umfassen. Die Auswerteeinrichtung 14 kann dabei einen Computer mit einer Speichereinrichtung umfassen. Auf der Speichereinrichtung kann ein Computerprogramm zur Überprüfung des Herstellungsprozesses von Verklebungen gespeichert sein. Dabei können insbesondere Verklebungen einer ersten Papierbahn 24 mit mindestens einer zweiten Papierbahn (25, 26) zu einer Produktbahn (28) überprüft werden. Das Computerprogramm kann dabei ausgelegt sein die folgenden Schritte auszuführen:
- Detektieren thermischer Strahlung der Produktbahn 28 nach dem Verkleben der ersten Papierbahn 24 mit der mindestens zweiten Papierbahn (25, 26),
- Erstellen thermografischer Daten 30 basierend auf der thermischen Strahlung (siehe **Fig. 2c** und **Fig. 3c**),
- Auswerten der thermografischen Daten 30 (siehe **Fig. 3** - **Fig. 5**), und
- Beurteilen der Verklebegüte der Papierverklebung anhand der Auswertung.
Durch diese besonders vorteilhaften Ausgestaltungen kann eine automatisierte Qualitätsüberprüfung bereitgestellt werden. Weiterhin kann durch die automatisierte Überprüfung eine 100% Kontrolle der Verklebungen sichergestellt werden. Durch die automatisierte und frühe Erkennung eventueller Fehler kurz nach der Klebestation 22 lässt sich der Herstellungsprozess bzw. der Verklebeprozess zügig anpassen, wodurch der Ausschuss fehlerhafter Erzeugnisse massiv reduziert werden kann im Vergleich zu einer Qualitätskontrolle zu einem späteren Zeitpunkt im Produktionsprozess, beispielsweise im Warenausgang.

In Ausgestaltungen, die mit der vorangehenden Ausgestaltung kombinierbar sind, kann das Detektieren ein Detektieren elektromagnetischer Strahlung im Infrarot-Bereich umfassen. Alternativ oder zusätzlich kann die thermische Strahlung der Produktbahn 28 in einem Erfassungsbereich 31, der sich zeilenförmig 31b in y-Richtung der Produktbahn 28 erstreckt, detektiert werden. Alternativ oder zusätzlich kann die thermische Strahlung der Produktbahn 28 in einem Erfassungsbereich 31, der sich zweidimensional 31a in x- und y-Richtung der Produktbahn 28 erstreckt, detektiert werden. Alternativ oder zusätzlich können die thermografischen Daten 30 in einem Auswertebereich 33, der vorzugsweise ein zweidimensionaler Datenbereich in x-Richtung 94 und y-Richtung 92 ist, erstellt werden. Alternativ oder zusätzlich kann die Beurteilung der Verklebegüte anhand einer geometrischen Auswertung der thermografischen Daten 30 erfolgen.

Diesbezüglich zeigt die **Fig. 2b** die Temperaturverteilungen einer fehlerfreien Wellpappe 28 entlang eines ersten beispielhaften Messbereichs 40 und eines zweiten beispielhaften Messbereichs 42 innerhalb des Erfassungsbereichs 31a aus **Fig. 2a****.** Die Kurven der Temperaturverteilungen der Messbereiche (40, 42) in **Fig. 2b** zeigen dabei jeweils den Temperaturverlauf 96a der Wellpappenoberfläche in x-Richtung 94 innerhalb des Erfassungsbereichs 31a über je drei Wellen (25a, 25b, 25c) der Wellpappe 28 an jeweils einer beispielhaften Position auf der y-Achse 92. Wie bereits weiter oben erwähnt, ist der **Fig. 2b** zu entnehmen, dass die Temperatur auf der Oberfläche der Wellpappe 28, also in dem gezeigten Ausführungsbeispiel die Oberflächentemperatur der flachen Papierbahn 24 im Kontaktbereich mit der mindestens zweiten Papierbahn 25, in diesem Fall die gewellte Papierbahn 25, am höchsten ist. Die Temperaturmaxima im jeweiligen Messbereiche (40, 42) sind durch die Strichlinien 40m und 42m angedeutet. Weiterhin ist der beispielhaften Temperaturverteilung (40a, 42a) der **Fig. 2b** zu entnehmen, dass die Wellpappe 28 in Richtung des Randbereichs, also in Transportrichtung 94a blickend, rechts und links am Rand der Bahnbreite 92a geringere Temperaturen aufweist als in der Mitte der Bahn (siehe **Fig. 2c**). Dies wird insbesondere bei Betrachtung der **Fig. 2c** deutlich, welche einen zum Erfassungsbereich 31a korrespondierenden Auswertebereich 33 mit beispielhaften Isothermen 32a zeigt. Es ist zu erkennen, dass die thermografischen Daten 30 der fehlerfreien Wellpappenverklebung ein charakteristisches Muster 32 aufweisen. Beispielsweise entspricht die Ausdehnung der Isotherme bei der Temperatur 42m in x-Richtung 94 für den Messbereich 40 der charakteristischen Strecke 44a und für den Messbereich 42 der charakteristischen Strecke 46a (jeweils im Bereich der Welle 25a gemessen).

Im Vergleich hierzu zeigen die **Fig. 3a** - **Fig. 3c** eine fehlerbehaftete Wellpappenverklebung mit korrespondierenden thermografischen Daten 330. Beispielhaft sind in der **Fig. 3a** drei flächige Fehlstellen (35a, 35b, 35c) dargestellt. In alternativen Ausführungen können aber auch andere Arten von Fehlstellen, insbesondere Fehlstellen im Randbereich der Produktbahn 28 und/oder Fehlstellen über die gesamte Bahnbreite 92a erfasst werden. Wie in den **Fig. 2a** - **Fig. 2c** sind in den **Fig. 3a** - **Fig. 3c** wieder zwei beispielhafte Messbereiche (340, 342) und Temperaturverteilungen (340a, 342a) gezeigt. Die Fehlstelle 35a überschneidet sich dabei mit Messbereich 342 und dort genauer mit der ersten Welle 25a und ist in der **Fig. 3b** an dem niedrigerem Temperaturmaximum 342n zu erkennen. In den thermografischen Daten 330 der **Fig. 3c** ist die Fehlstelle 35a als geometrische Inhomogenität 334 zu erkennen. Da die maximale Temperatur 342n am Ort der Fehlstelle 35a geringer ist als an einer fehlerfreien Stelle (vgl. beispielsweise mit 342m bei Welle 25b in **Fig. 3b** oder mit 42m bei Welle 25a in **Fig. 2b**) ist von einer Fehlstelle 35 aufgrund von Ablösung der zu verklebenden Papierbahnen (24, 25, 26) und/oder einem zu geringen Klebemittelauftrag in der Klebestation 22 auszugehen. Wäre die Fehlstelle 35c durch einen zu großen Klebemittelauftrag bedingt, so würde dies in einem größeren Maximum 342n als Maximum 342m einer fehlerfreien Verklebung und/oder in einer größeren Strecke 346a als die Strecke 346b einer fehlerfreien Verklebung resultieren (siehe **Fig. 3a** und **Fig. 3b**). Ebenso würde die Inhomogenität 334 in letztgenanntem Fall eine größere Ausdehnung in x-Richtung 94 aufweisen als die Isothermen 332a im Bereich der Wellen 25b und 25c an einer anderen Position des Messbereichs 342 in x-Richtung 94.

Durch derartige vorteilhafte Ausgestaltungen kann nicht nur überprüft werden, ob der Klebemittelauftrag ausreichend oder zu umfangreich war, sondern es kann auch unabhängig vom Klebemittelauftrag überprüft werden, ob eine erfolgreiche Verklebung zustande gekommen ist. Das heißt es kann überprüft werden, ob die zu verklebenden Papierbahnen (24, 25, 26) nach dem Klebemittelauftrag und nach dem Inkontaktbringen auch in Kontakt geblieben sind. Dies ist deshalb möglich, da die zu verklebenden Papierbahnen (24, 25, 26) bzw. auch deren Oberflächen eine bestimmte Temperaturverteilung (40a, 42a, 340a, 342a) und dementsprechend auch spezifisches thermografische Eigenschaften auf ihren Oberflächen aufweisen, anhand derer Rückschlüsse auf den Verklebezustand gezogen werden können.

Das heißt, Fehlstellen 35 der Verklebung können durch geometrische Inhomogenitäten 334 in den thermografischen Daten (30, 330) detektiert werden. Weiterhin können Fehlstellen 35 anhand geometrischer Inhomogenitäten 334, die von einem vorgegebenen Muster (32, 332) in den thermografischen Daten (30, 330) abweichen, identifiziert werden. Weiterhin können die Fehlstellen anhand einer Temperaturabweichung von einer fehlerfreien Verklebung detektiert werden. Als Indikator für eine Anomalie oder Fehlstelle kann beispielsweise eine Temperaturabweichung von mehr als 3°C, insbesondere von mehr als 5°C dienen. Eine Kombination aus Temperaturabweichung und geometrischer Ausprägung lässt eine Klassifizierung der jeweiligen Fehlstelle oder eines Events zu. Zu diesen geometrischen Inhomogenitäten 334 gehören insbesondere kreisförmige, rechteckige und/oder im Wesentlichen über die gesamte Bahnbreite 92a in y-Richtung 92 verlaufende geometrische Inhomogenitäten. Mit anderen Worten bedeutet dies, dass anhand der erfassten thermischen Strahlung, thermografische Daten (30, 330, 430, 530) erstellt werden können, auf deren Basis die Auswertung und Beurteilung abläuft.

Wie in Zusammenhang mit der **Fig. 3b** bereits erwähnt, kann die Auswertung eine ortsaufgelöste Bestimmung von geometrischen Dimensionen einer oder mehrerer isothermer Bereiche (32a, 332a) und/oder Teilbereiche in den thermografischen Daten (30, 330) umfassen. Die geometrischen Dimensionen des einen oder der mehreren isothermen Bereiche (32a, 332a) und/oder Teilbereiche kann dabei bevorzugt die Länge in x-Richtung 94 und/oder die Breite in y-Richtung 92 umfassen. Beispielhaft wurde bereits in Zusammenhang mit den **Fig. 2b** und **Fig. 3b** eine Auswertung der Länge der isothermen Teilbereiche in x-Richtung 94 erläutert. Dabei kann die Verklebegüte anhand eines Vergleichs der geometrischen Dimensionen des einen oder der mehreren isothermen Bereiche (32a, 332a) und/oder Teilbereiche einer Verklebung mit Referenzwerten der geometrischen Dimensionen beurteilt werden. Im Ausführungsbeispiel der **Fig. 3b** könnte beispielsweise durch einen Vergleich der Länge 346a mit der Länge 346b einer fehlerfreien Verklebung an einer korrespondierenden Position in y-Richtung 92 einer Isotherme, überprüft werden, ob die Verklebung fehlerfrei ist. Als Referenzwert kann dabei sowohl ein vorbestimmter Referenzwert, als auch ein Referenzwert, der über einen Durchschnitt der vorherig überprüften Verklebungen gebildet wurde, dienen. Insbesondere können die Referenzwerte dabei über einen Durchschnitt der zwei vorherig überprüften Verklebungen, bevorzugt der fünf vorherig überprüften Verklebungen und besonders bevorzugt der mindestens zehn vorherig überprüften Verklebungen gebildet werden. Alternativ kann der Referenzwert auch von einem Anlagenbediener manuell eingegeben oder abgeändert werden. Eine Verklebung kann dabei als fehlerfrei, beziehungsweise die Verklebegüte kann als ausreichend beurteilt werden, wenn der jeweilige Vergleich eine maximale Abweichung von 0% bis 50%, bevorzugt von 0% bis 20% und besonders bevorzugt von 0% bis 5% ergibt.

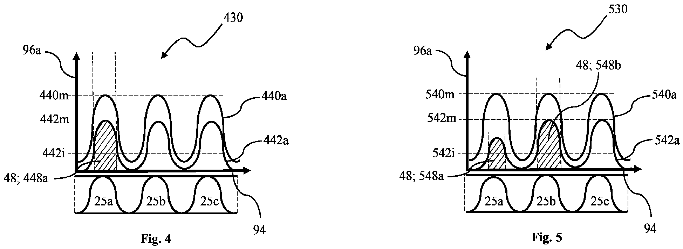

Alternativ oder zusätzlich kann die Auswertung die Bildung eines Integrals 48 über einen zeilenförmigen Teilbereich der thermografischen Daten (30, 330, 430, 530) einer oder mehrerer isothermer Bereiche (32a, 332a) und/oder Teilbereiche in x-Richtung 94 umfassen. Dabei kann die Verklebegüte anhand eines Vergleichs des Integrals 48 des einen oder der mehreren isothermen Bereiche (32a, 332a) und/oder Teilbereiche einer Verklebung mit Referenzwerten des jeweiligen Integrals 48 beurteilt werden. Diesbezüglich zeigen **Fig. 4** und **Fig. 5** jeweils eine den **Fig. 2b** bzw. **Fig. 3b** entsprechende thermografische Darstellung, allerdings mit integraler Auswertung. Zu erkennen sind in der **Fig. 4** wieder die Temperaturverteilungen (440a, 442a) für die beiden Messbereiche (40, 42) einer fehlerfreien Verklebung aus **Fig. 2a****.** Beispielhaft zeigt die **Fig. 4** im Bereich der Welle 25a ein Integral 448a für eine Vergleichstemperatur 442i, die zur Begrenzung des Integrationsbereichs dient. Die **Fig. 5** zeigt hingegen die Temperaturverteilungen (540a, 542a) für die beiden Messbereiche (340, 342) einer fehlerbehafteten Verklebung aus **Fig. 3a****.** Ebenso wie in **Fig. 4**, ist in **Fig. 5** eine Vergleichstemperatur 542i, die zur Begrenzung des Integrationsbereichs dient eingezeichnet. Durch die Fehlstelle 35a (siehe **Fig. 3a**) im Bereich der Welle 25a des Messbereichs 342 ist die Temperatur 96a und somit auch das Integral 548a im Bereich der Welle 25a des Messbereichs 342 wesentlich kleiner als ein Integral in einem fehlerfreien Bereich (siehe Integrale 448a und 548b).

Alternativ oder zusätzlich kann die Auswertung die Bildung eines Flächenintegrals über einen Teilbereich der thermografischen Daten (30, 330, 430, 530) einer oder mehrerer isothermer Bereiche (32a, 332a) und/oder Teilbereiche in x-Richtung 94 und y-Richtung 92 umfassen. Hierbei kann die Verklebegüte anhand eines Vergleichs des Flächenintegrals des einen oder der mehreren isothermen Bereiche (32a, 332a) und/oder Teilbereiche einer Verklebung mit Referenzwerten des jeweiligen Flächenintegrals beurteilt werden. Die Referenzwerte für den Vergleich mit Integral in x-Richtung 94 und/oder dem Flächenintegral in x-Richtung 94 und y-Richtung 92 können dabei vorbestimmte Referenzwerte umfassen. Alternativ oder zusätzlich können die Referenzwerte auch über einen Durchschnitt der vorherig überprüften Verklebungen gebildet werden. Insbesondere können die Referenzwerte dabei über einen Durchschnitt der zwei vorherig überprüften Verklebungen, bevorzugt der fünf vorherig überprüften Verklebungen und besonders bevorzugt der mindestens zehn vorherig überprüften Verklebungen gebildet werden. Alternativ kann der Referenzwert auch von einem Anlagenbediener manuell eingegeben oder abgeändert werden. Eine Verklebung kann dabei als fehlerfrei, beziehungsweise die Verklebegüte kann als ausreichend beurteilt werden, wenn der jeweilige Vergleich eine maximale Abweichung von 0% bis 50%, bevorzugt von 0% bis 20% und besonders bevorzugt von 0% bis 5% ergibt.

Alternativ oder zusätzlich kann die Auswertung auch einen Vergleich der thermografischen Daten (30, 330, 430, 530) mit einem thermografischen Masterbild umfassen. Das thermografische Masterbild (golden template) stellt dabei das einer perfekten Verklebung entsprechende thermografische Bild beziehungsweise die einer perfekten Verklebung entsprechende thermografischen Daten dar.

Weiterhin kann das Detektieren der thermischen Strahlung mit einer Infrarot-Kamera 12 durchgeführt werden (siehe **Fig. 1**).

Weiterhin kann das Computerprogramm ausgelegt sein, in-line beim Herstellungsprozess von Papierverklebungen, vorzugsweise Wellpappenverklebungen ausgeführt zu werden.

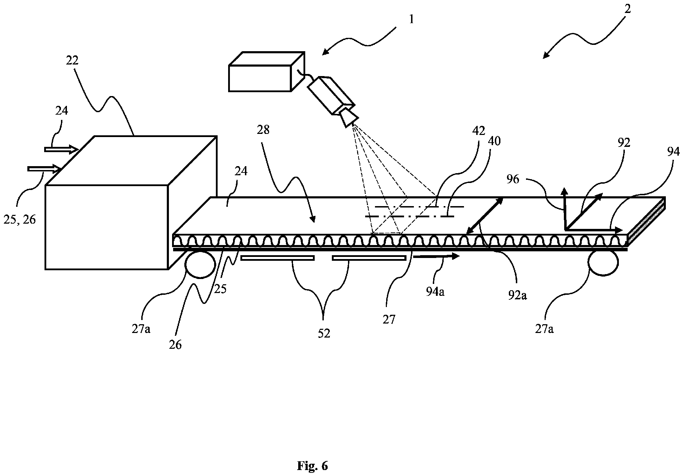

Wie in **Fig. 6** dargestellt, umfasst die Erfindung weiterhin eine Wellpappenmaschine 2. Die Wellpappenmaschine 2 umfasst eine Transporteinrichtung 27 zum Transport von Wellpappe 28 in einer Transportrichtung 94a. Die Transporteinrichtung 27 kann dabei ein Förderband mit Rollen 27a oder einfach nur Förderrollen 27a umfassen. Weiterhin umfasst die Wellpappenmaschine 2 eine Klebestation 22, die zum Verkleben einer ersten Papierbahn 24 mit mindestens einer zweiten Papierbahn (25, 26) ausgelegt ist. Weiterhin umfasst die Wellpappenmaschine 2 ein Messsystem zur Überprüfung des Herstellungsprozesses der Wellpappe 28. Das Messsystem kann dabei ein Messsystem 1 nach irgendeiner der vorhergehen Ausgestaltungen sein.

Weiterhin kann die Wellpappenmaschine 2 Heizelemente 52 umfassen, die an der Transporteinrichtung 27 angeordnet sind. Alternativ und insbesondere wenn die Transporteinrichtung 27 nur Förderrollen 27a umfasst, können die Heizelemente 52 auch direkt an der Wellpappe 28, beispielsweise in negativer z-Richtung 96 unter der Wellpappe 28 mit einem Luftspalt angeordnet sein. Alternativ können die Heizelemente 52 auch an einer anderen Position in x-Richtung 94 und/oder auf der anderen Seite der Wellpappenbahn 28 in positiver z-Richtung 96 angeordnet sein.

Weiterhin kann die Wellpappenmaschine 2 eine Ausschusseinrichtung umfassen, die hier nicht dargestellt ist. Die Ausschusseinrichtung kann dabei in Transportrichtung 94a hinter dem Messsystem 1 angeordnet sein. Weiterhin kann die Ausschusseinrichtung ausgelegt sein, Wellpappe mit einer Verklebegüte unterhalb eines Grenzwerts auszusortieren.

In Ausgestaltungen der erfindungsgemäßen Wellpappenmaschine 2, die mit irgendeiner der drei vorangehenden Ausgestaltungen kombinierbar sind, kann das Messsystem 1 auf eine Oberflächenseite der ersten Papierbahn 24 gerichtet sein, deren Normalenvektor in die entgegengesetzte Richtung der mindestens zweiten Papierbahn (25, 26) gerichtet ist. Alternativ oder zusätzlich kann das Messsystem 1 auf eine Oberflächenseite der mindestens zweiten Papierbahn (25, 26) gerichtet sein, deren Normalenvektor in die entgegengesetzte Richtung der ersten Papierbahn 24 gerichtet ist. Das bedeutet, dass die Messeinrichtung 1 lediglich auf eine Oberfläche einer Produktbahn 28 gerichtet sein muss und somit auch nur auf einer Oberflächenseite einer Produktbahn 28 angeordnet sein braucht. Alternativ kann die Messeinrichtung 1 aber auch zwei Einheiten umfassen, die auf beiden Seiten der Produktbahn 28 angeordnet sind, um beide Oberflächen der Produktbahn 28 zu erfassen. Im Beispiel der **Fig. 6** wird eine obere, flache Papierbahn 24 und eine untere, flache Papierbahn 26 mit einer dazwischen angeordneten gewellten Papierbahn 25 verklebt. Da die Transporteinrichtung 27, wie bereits erwähnt, auch nur Förderrollen 27a umfassen könnte, könnte durch ein Messsystem 1 mit einer zweiten Messeinrichtung (nicht dargestellt), welche auf die Oberfläche der unteren Papierbahn 26 gerichtet ist, auch die Verklebung der unteren, flachen Papierbahn 26 mit der gewellten Papierbahn 25 überprüft werden. Alternativ kann das Messsystem 1 auch nur auf die Oberfläche der unteren Papierbahn 26 gerichtet sein, um die Verklebung der unteren, flachen Papierbahn 26 mit der gewellten Papierbahn 25 zu überprüfen. Alternativ, insbesondere in Ausführungsformen zur Herstellung einer einseitigen Wellpappe 28, kann das Messsystem 1 auch auf die Oberfläche der gewellten Papierbahn 25 gerichtet sein.

Die Erfindung umfasst weiterhin ein Verfahren zur Überprüfung des Herstellungsprozesses von Verklebungen einer ersten Papierbahn 24 mit mindestens einer zweiten Papierbahn (25, 26) zu einer Produktbahn (28), unter Verwendung eines Messsystems. Das Verfahren umfasst dabei die folgenden Schritte:
- Detektieren thermischer Strahlung der Produktbahn 28 nach dem Verkleben der ersten Papierbahn 24 mit der mindestens zweiten Papierbahn (25, 26),
- Erstellen thermografischer Daten (30, 330) basierend auf der thermischen Strahlung (siehe **Fig. 2c** und **Fig. 3c**),
- Auswerten der thermografischen Daten (30, 330, 430, 530) (siehe **Fig. 3** - **Fig. 5**), und
- Beurteilen der Verklebegüte der Papierverklebung anhand der Auswertung.
Durch diese besonders vorteilhaften Ausgestaltungen kann eine automatisierte Qualitätsüberprüfung bereitgestellt werden. Weiterhin kann durch die automatisierte Überprüfung eine 100% Kontrolle der Verklebungen sichergestellt werden. Durch die automatisierte und frühe Erkennung eventueller Fehler kurz nach der Klebestation 22 lässt sich der Herstellungsprozess bzw. der Verklebeprozess zügig anpassen, wodurch der Ausschuss fehlerhafter Erzeugnisse massiv reduziert werden kann im Vergleich zu einer Qualitätskontrolle zu einem späteren Zeitpunkt im Produktionsprozess, beispielsweise im Warenausgang.

In Ausgestaltungen, die mit der vorangehenden Ausgestaltung kombinierbar sind, kann das Detektieren ein Detektieren elektromagnetischer Strahlung im Infrarot-Bereich umfassen. Alternativ oder zusätzlich kann die thermische Strahlung der Produktbahn 28 in einem Erfassungsbereich 31, der sich zeilenförmig 31b in y-Richtung der Produktbahn 28 erstreckt, detektiert werden. Alternativ oder zusätzlich kann die thermische Strahlung der Produktbahn 28 in einem Erfassungsbereich 31, der sich zweidimensional 31a in x- und y-Richtung der Produktbahn 28 erstreckt, detektiert werden. Alternativ oder zusätzlich können die thermografischen Daten 30 in einem Auswertebereich 33, der vorzugsweise ein zweidimensionaler Datenbereich in x-Richtung 94 und y-Richtung 92 ist, erstellt werden. Alternativ oder zusätzlich kann die Beurteilung der Verklebegüte anhand einer geometrischen Auswertung der thermografischen Daten 30 erfolgen.

Durch derartige vorteilhafte Ausgestaltungen kann nicht nur überprüft werden, ob der Klebemittelauftrag ausreichend oder zu umfangreich war, sondern es kann auch unabhängig vom Klebemittelauftrag überprüft werden, ob eine erfolgreiche Verklebung zustande gekommen ist. Das heißt es kann überprüft werden, ob die zu verklebenden Papierbahnen (24, 25, 26) nach dem Klebemittelauftrag und nach dem Inkontaktbringen auch in Kontakt geblieben sind. Dies ist deshalb möglich, da die zu verklebenden Papierbahnen (24, 25, 26) bzw. auch deren Oberflächen eine bestimmte Temperaturverteilung (40a, 42a, 340a, 342a) und dementsprechend auch spezifisches thermografische Eigenschaften auf ihren Oberflächen aufweisen, anhand derer Rückschlüsse auf den Verklebezustand gezogen werden können.

Das heißt, Fehlstellen 35 der Verklebung können durch geometrische Inhomogenitäten 334 in den thermografischen Daten (30, 330) detektiert werden. Weiterhin können Fehlstellen 35 anhand geometrischer Inhomogenitäten 334, die von einem vorgegebenen Muster (32, 332) in den thermografischen Daten (30, 330) abweichen, identifiziert werden. Zu diesen geometrischen Inhomogenitäten 334 gehören insbesondere kreisförmige, rechteckige und/oder im Wesentlichen über die gesamte Bahnbreite 92a in y-Richtung 92 verlaufende geometrische Inhomogenitäten. Mit anderen Worten bedeutet dies, dass anhand der erfassten thermischen Strahlung, thermografische Daten (30, 330, 430, 530) erstellt werden können, auf deren Basis die Auswertung und Beurteilung abläuft.

Wie in Zusammenhang mit der **Fig. 3b** bereits erwähnt, kann die Auswertung eine ortsaufgelöste Bestimmung von geometrischen Dimensionen einer oder mehrerer isothermer Bereiche (32a, 332a) und/oder Teilbereiche in den thermografischen Daten (30, 330) umfassen. Die geometrischen Dimensionen des einen oder der mehreren isothermen Bereiche (32a, 332a) und/oder Teilbereiche kann dabei bevorzugt die Länge in x-Richtung 94 und/oder die Breite in y-Richtung 92 umfassen. Beispielhaft wurde bereits in Zusammenhang mit den **Fig. 2b** und **Fig. 3b** eine Auswertung der Länge der isothermen Teilbereiche in x-Richtung 94 erläutert. Dabei kann die Verklebegüte anhand eines Vergleichs der geometrischen Dimensionen des einen oder der mehreren isothermen Bereiche (32a, 332a) und/oder Teilbereiche einer Verklebung mit Referenzwerten der geometrischen Dimensionen beurteilt werden. Im Ausführungsbeispiel der **Fig. 3b** könnte beispielsweise durch einen Vergleich der Länge 346a mit der Länge 346b einer fehlerfreien Verklebung an einer korrespondierenden Position in y-Richtung 92 einer Isotherme, überprüft werden, ob die Verklebung fehlerfrei ist. Als Referenzwert kann dabei sowohl ein vorbestimmter Referenzwert, als auch ein Referenzwert, der über einen Durchschnitt der vorherig überprüften Verklebungen gebildet wurde, dienen. Insbesondere können die Referenzwerte dabei über einen Durchschnitt der zwei vorherig überprüften Verklebungen, bevorzugt der fünf vorherig überprüften Verklebungen und besonders bevorzugt der mindestens zehn vorherig überprüften Verklebungen gebildet werden. Alternativ kann der Referenzwert auch von einem Anlagenbediener manuell eingegeben oder abgeändert werden. Eine Verklebung kann dabei als fehlerfrei, beziehungsweise die Verklebegüte kann als ausreichend beurteilt werden, wenn der jeweilige Vergleich eine maximale Abweichung von 0% bis 50%, bevorzugt von 0% bis 20% und besonders bevorzugt von 0% bis 5% ergibt.

Alternativ oder zusätzlich kann die Auswertung die Bildung eines Integrals 48 über einen zeilenförmigen Teilbereich der thermografischen Daten (30, 330, 430, 530) einer oder mehrerer isothermer Bereiche (32a, 332a) und/oder Teilbereiche in x-Richtung 94 umfassen. Dabei kann die Verklebegüte anhand eines Vergleichs des Integrals 48 des einen oder der mehreren isothermen Bereiche (32a, 332a) und/oder Teilbereiche einer Verklebung mit Referenzwerten des jeweiligen Integrals 48 beurteilt werden. Diesbezüglich zeigen **Fig. 4** und **Fig. 5** jeweils eine den **Fig. 2b** bzw. **Fig. 3b** entsprechende thermografische Darstellung, allerdings mit integraler Auswertung. Zu erkennen sind in der **Fig. 4** wieder die Temperaturverteilungen (440a, 442a) für die beiden Messbereiche (40, 42) einer fehlerfreien Verklebung aus **Fig. 2a****.** Beispielhaft zeigt die **Fig. 4** im Bereich der Welle 25a ein Integral 448a für eine Vergleichstemperatur 442i, die zur Begrenzung des Integrationsbereichs dient. Die **Fig. 5** zeigt hingegen die Temperaturverteilungen (540a, 542a) für die beiden Messbereiche (340, 342) einer fehlerbehafteten Verklebung aus **Fig. 3a****.** Ebenso wie in **Fig. 4**, ist in **Fig. 5** eine Vergleichstemperatur 542i, die zur Begrenzung des Integrationsbereichs dient eingezeichnet. Durch die Fehlstelle 35a (siehe **Fig. 3a**) im Bereich der Welle 25a des Messbereichs 342 ist die Temperatur 96a und somit auch das Integral 548a im Bereich der Welle 25a des Messbereichs 342 wesentlich kleiner als ein Integral in einem fehlerfreien Bereich (siehe Integrale 448a und 548b). Alternativ oder zusätzlich kann die Auswertung die Bildung eines Flächenintegrals über einen Teilbereich der thermografischen Daten (30, 330, 430, 530) einer oder mehrerer isothermer Bereiche (32a, 332a) und/oder Teilbereiche in x-Richtung 94 und y-Richtung 92 umfassen. Hierbei kann die Verklebegüte anhand eines Vergleichs des Flächenintegrals des einen oder der mehreren isothermen Bereiche (32a, 332a) und/oder Teilbereiche einer Verklebung mit Referenzwerten des jeweiligen Flächenintegrals beurteilt werden. Die Referenzwerte für den Vergleich mit Integral in x-Richtung 94 und/oder dem Flächenintegral in x-Richtung 94 und y-Richtung 92 können dabei vorbestimmte Referenzwerte umfassen. Alternativ oder zusätzlich können die Referenzwerte auch über einen Durchschnitt der vorherig überprüften Verklebungen gebildet werden. Insbesondere können die Referenzwerte dabei über einen Durchschnitt der zwei vorherig überprüften Verklebungen, bevorzugt der fünf vorherig überprüften Verklebungen und besonders bevorzugt der mindestens zehn vorherig überprüften Verklebungen gebildet werden. Alternativ kann der Referenzwert auch von einem Anlagenbediener manuell eingegeben oder abgeändert werden. Eine Verklebung kann dabei als fehlerfrei, beziehungsweise die Verklebegüte kann als ausreichend beurteilt werden, wenn der jeweilige Vergleich eine maximale Abweichung von 0% bis 50%, bevorzugt von 0% bis 20% und besonders bevorzugt von 0% bis 5% ergibt.

Weiterhin kann das Detektieren der thermischen Strahlung mit einer Infrarot-Kamera 12 durchgeführt werden (siehe **Fig. 1**).

Weiterhin kann das Computerprogramm ausgelegt sein, in-line beim Herstellungsprozess von Papierverklebungen, vorzugsweise Wellpappenverklebungen ausgeführt zu werden.

Weiterhin kann die thermische Strahlung von einer Oberflächenseite der ersten Papierbahn 24 detektiert werden, deren Normalenvektor in die entgegengesetzte Richtung der mindestens zweiten Papierbahn (25, 26) gerichtet ist. Alternativ oder zusätzlich kann die thermische Strahlung von einer Oberflächenseite der mindestens zweiten Papierbahn (25, 26) detektiert werden, deren Normalenvektor in die entgegengesetzte Richtung der ersten Papierbahn 24 gerichtet ist.

In Ausgestaltungen des erfindungsgemäßen Verfahrens kann die Papierverklebung insbesondere eine Wellpappenverklebung sein.

In Ausgestaltungen des erfindungsgemäßen Verfahrens, kann die Produktbahn insbesondere eine Wellpappenbahn 28 sein.

In Ausgestaltungen des erfindungsgemäßen Verfahrens, kann das Verfahren mit einem Messsystem 1 gemäß irgendeiner vorangehenden Ausführungen durchgeführt werden.

Obwohl die vorliegende Erfindung oben beschrieben wurde und in den beigefügten Ansprüchen definiert ist, sollte verstanden werden, dass die Erfindung alternativ auch entsprechend der folgenden Ausführungsformen definiert werden kann:
1. Messsystem zur Verwendung in einer Wellpappenmaschine, wobei das Messsystem eine Messeinrichtung umfasst;
   dadurch gekennzeichnet, dass
   die Messeinrichtung elektromagnetische Wellen im Infrarot-Bereich erfasst und auf einen Bereich einer Transporteinrichtung der Wellpappenmaschine in Transportrichtung hinter einer Klebestation der Wellpappenmaschine gerichtet ist.
2. Messsystem nach Ausführungsform 1, dadurch gekennzeichnet, dass die Messeinrichtung ausgelegt ist, die Temperatur der Wellpappe zu erfassen.
3. Messsystem nach Ausführungsform 2, dadurch gekennzeichnet, dass die Messeinrichtung vorzugsweise eine bildgebende Messeinrichtung umfasst.
4. Messsystem nach einer der Ausführungsformen 2 oder 3, dadurch gekennzeichnet, dass die Messeinrichtung ausgelegt ist, eine Temperaturverteilung thermografisch darzustellen und zu analysieren.
5. Messsystem nach einer der vorangehenden Ausführungsformen, dadurch gekennzeichnet, dass die Messeinrichtung ausgelegt ist, die Temperatur, vorzugsweise die Oberflächentemperatur der Wellpappe zu erfassen.
6. Messsystem nach einer der vorangehenden Ausführungsformen, dadurch gekennzeichnet, dass die Messeinrichtung ausgelegt ist eine Temperaturverteilung der Wellpappe zu beurteilen.
7. Messsystem nach einer der vorangehenden Ausführungsformen, dadurch gekennzeichnet, dass die Messeinrichtung ausgelegt ist eine Temperaturverteilung der Wellpappe, vorzugsweise eine Temperaturverteilung der Wellpappe entlang der Bahnbreite zu erfassen.
8. Messsystem nach einer der vorangehenden Ausführungsformen, dadurch gekennzeichnet, dass die Messeinrichtung ausgelegt ist, eine Temperaturverteilung der Wellpappe zeilenförmig und/oder matrixförmig zu beurteilen.
9. Messsystem nach einer der vorangehenden Ausführungsformen, dadurch gekennzeichnet, dass die Messeinrichtung ausgelegt ist, die Verklebegüte der Wellpappe, vorzugsweise anhand einer Temperaturverteilung der Wellpappe zu beurteilen.
10. Messsystem nach einer der vorangehenden Ausführungsformen, dadurch gekennzeichnet, dass die Messeinrichtung eine Infrarot-Kamera umfasst.
11. Messsystem nach einer der vorangehenden Ausführungsformen, dadurch gekennzeichnet, dass die Messeinrichtung eine Auswerteeinrichtung zur Auswertung der erfassten elektromagnetischen Wellen im Infrarot-Bereich umfasst.
12. Messsystem nach Ausführungsform 11, dadurch gekennzeichnet, dass die Auswerteeinrichtung einen Computer mit einer Speichereinrichtung umfasst, wobei auf der Speichereinrichtung ein Computerprogramm zur Überprüfung des Herstellungsprozesses von Verklebungen einer ersten Papierbahn mit mindestens einer zweiten Papierbahn zu einer Produktbahn gespeichert ist, das ausgelegt ist, die folgenden Schritte auszuführen:
   - Detektieren thermischer Strahlung der Produktbahn nach dem Verkleben der ersten Papierbahn mit der mindestens zweiten Papierbahn,
   - Erstellen thermografischer Daten basierend auf der thermischen Strahlung,
   - Auswerten der thermografischen Daten, und
   - Beurteilen der Verklebegüte der Papierverklebung anhand der Auswertung.
13. Messsystem nach Ausführungsform 12, wobei das Detektieren ein Detektieren elektromagnetischer Strahlung im Infrarot-Bereich umfasst.
14. Messsystem nach einer der vorangehenden Aus führungs formen 12 oder 13, wobei die thermische Strahlung der Produktbahn in einem Erfassungsbereich, der sich zeilenförmig in y-Richtung der Produktbahn und/oder zweidimensional in x- und y-Richtung der Produktbahn erstreckt, detektiert wird.
15. Messsystem nach einer der vorangehenden Ausführungsformen 12 bis 14, wobei die thermografischen Daten in einem Auswertebereich, der vorzugsweise ein zweidimensionaler Datenbereich in x- und y-Richtung ist, erstellt werden.
16. Messsystem nach einer der vorangehenden Aus führungs formen 12 bis 15, wobei die Beurteilung der Verklebegüte anhand einer geometrischen Auswertung der thermografischen Daten erfolgt.
17. Messsystem nach einer der vorangehenden Ausführungsformen 12 bis 16, wobei Fehlstellen der Verklebung durch geometrische Inhomogenitäten in den thermografischen Daten detektiert werden.
18. Messsystem nach Ausführungsform 17, wobei die Fehlstellen, insbesondere anhand kreisförmiger, rechteckiger und/oder im Wesentlichen über die gesamte Bahnbreite in y-Richtung verlaufender geometrischen Inhomogenitäten, die von einem vorgegebenen Muster in den thermografischen Daten abweichen, identifiziert werden.
19. Messsystem nach einer der Ausführungsformen 12 bis 18, wobei die Auswertung eine ortsaufgelöste Bestimmung der geometrischen Dimensionen, bevorzugt der Länge in x-Richtung und/oder der Breite in y-Richtung, einer oder mehrerer isothermer Bereiche und/oder Teilbereiche in den thermografischen Daten umfasst.
20. Messsystem nach Ausführungsform 19, wobei die Verklebegüte anhand eines Vergleichs der geometrischen Dimensionen des einen oder der mehreren isothermen Bereiche und/oder Teilbereiche einer Verklebung mit Referenzwerten der geometrischen Dimensionen beurteilt wird.
21. Messsystem nach einer der Ausführungsformen 12 bis 18, wobei die Auswertung die Bildung eines Integrals über einen zeilenförmigen Teilbereich der thermografischen Daten einer oder mehrerer isothermer Bereiche und/oder Teilbereiche in x-Richtung umfasst.
22. Messsystem nach Ausführungsform 21, wobei die Verklebegüte anhand eines Vergleichs des Integrals des einen oder der mehreren isothermen Bereiche und/oder Teilbereiche einer Verklebung mit Referenzwerten des jeweiligen Integrals beurteilt wird.
23. Messsystem nach einer der Ausführungsformen 12 bis 18, wobei die Auswertung die Bildung eines Flächenintegrals über einen Teilbereich der thermografischen Daten einer oder mehrerer isothermer Bereiche und/oder Teilbereiche in x-Richtung und y-Richtung umfasst.
24. Messsystem nach Ausführungsform 23, wobei die Verklebegüte anhand eines Vergleichs des Flächenintegrals des einen oder der mehreren isothermen Bereiche und/oder Teilbereiche einer Verklebung mit Referenzwerten des jeweiligen Flächenintegrals beurteilt wird.
25. Messsystem nach einer der Ausführungsformen 20, 22 oder 24, wobei die Referenzwerte, vorbestimmte Referenzwerte umfassen und/oder über einen Durchschnitt der vorherig überprüften Verklebungen gebildet werden, insbesondere der zwei vorherig überprüften Verklebungen, bevorzugt der fünf vorherig überprüften Verklebungen und besonders bevorzugt der mindestens zehn vorherig überprüften Verklebungen.
26. Messsystem nach einer der Ausführungsformen 20, 22, 24 oder 25, wobei die Verklebegüte ausreichend ist, wenn der jeweilige Vergleich eine maximale Abweichung von 0% bis 50%, bevorzugt von 0% bis 20% und besonders bevorzugt von 0% bis 5% ergibt.
27. Messsystem nach einer der vorangehenden Ausführungsformen 12 bis 26, wobei das Detektieren der thermischen Strahlung mit einer Infrarot-Kamera durchgeführt wird.
28. Messsystem nach einer der Ausführungsformen 12 bis 27, wobei das Computerprogramm ausgelegt ist, in-line beim Herstellungsprozess von Papierverklebungen, vorzugsweise Wellpappenverklebungen ausgeführt zu werden.
29. Wellpappenmaschine umfassend:
   eine Transporteinrichtung zum Transport von Wellpappe in einer Transportrichtung,
   eine Klebestation ausgelegt zum Verkleben einer ersten Papierbahn mit mindestens einer zweiten Papierbahn, und
   ein Messsystem zur Überprüfung des Herstellungsprozesses von Verklebungen der Wellpappe;
   dadurch gekennzeichnet, dass
   das Messsystem ein Messsystem nach einer der Ausführungsformen 1 bis 28 ist.
30. Wellpappenmaschine nach Ausführungsform 29, dadurch gekennzeichnet, dass die Wellpappenmaschine weiterhin Heizelemente umfasst, die an der Transporteinrichtung angeordnet sind.
31. Wellpappenmaschine nach einer der Ausführungsformen 29 oder 30, dadurch gekennzeichnet, dass die Wellpappenmaschine weiterhin eine Ausschusseinrichtung umfasst, die in Transportrichtung hinter dem Messsystem angeordnet ist und die ausgelegt ist, Wellpappe mit einer Verklebegüte unterhalb eines Grenzwerts auszusortieren.
32. Wellpappenmaschine nach einer der vorangehenden Ausführungsformen 29 bis 31, wobei das Messsystem auf eine Oberflächenseite der jeweils ersten und/oder der mindestens zweiten Papierbahn gerichtet ist, deren Normalenvektor in die entgegengesetzte Richtung der jeweils anderen Papierbahn gerichtet ist.
33. Verfahren zur Überprüfung des Herstellungsprozesses von Verklebungen einer ersten Papierbahn mit mindestens einer zweiten Papierbahn zu einer Produktbahn, unter Verwendung eines Messsystems,
   gekennzeichnet durch die folgenden Schritte:
   - Detektieren thermischer Strahlung der Produktbahn nach dem Verkleben der ersten Papierbahn mit der mindestens zweiten Papierbahn,
   - Erstellen thermografischer Daten basierend auf der thermischen Strahlung,
   - Auswerten der thermografischen Daten, und
   - Beurteilen der Verklebegüte der Papierverklebung anhand der Auswertung.
34. Verfahren nach Ausführungsform 33, wobei das Detektieren ein Detektieren elektromagnetischer Strahlung im Infrarot-Bereich umfasst.
35. Verfahren nach einer der vorangehenden Ausführungsformen 33 oder 34, wobei die thermische Strahlung der Produktbahn in einem Erfassungsbereich, der sich zeilenförmig in y-Richtung der Produktbahn und/oder zweidimensional in x- und y-Richtung der Produktbahn erstreckt, detektiert wird.
36. Verfahren nach einer der vorangehenden Ausführungsformen 33 bis 35, wobei die thermografischen Daten in einem Auswertebereich, der vorzugsweise ein zweidimensionaler Datenbereich in x- und y-Richtung ist, erstellt werden.
37. Verfahren nach einer der vorangehenden Ausführungsformen 33 bis 36, wobei die Beurteilung der Verklebegüte anhand einer geometrischen Auswertung der thermografischen Daten erfolgt.
38. Verfahren nach einer der vorangehenden Ausführungsformen 33 bis 37, wobei Fehlstellen der Verklebung durch geometrische Inhomogenitäten in den thermografischen Daten detektiert werden.
39. Verfahren nach Ausführungsform 38, wobei die Fehlstellen, insbesondere anhand kreisförmiger, rechteckiger und/oder im Wesentlichen über die gesamte Bahnbreite in y-Richtung verlaufender geometrischen Inhomogenitäten, die von einem vorgegebenen Muster in den thermografischen Daten abweichen, identifiziert werden.
40. Verfahren nach einer der Ausführungsformen 33 bis 39, wobei die Auswertung eine ortsaufgelöste Bestimmung der geometrischen Dimensionen, bevorzugt der Länge in x-Richtung und/oder der Breite in y-Richtung, einer oder mehrerer isothermer Bereiche und/oder Teilbereiche in den thermografischen Daten umfasst.
41. Verfahren nach Ausführungsform 40, wobei die Verklebegüte anhand eines Vergleichs der geometrischen Dimensionen des einen oder der mehreren isothermen Bereiche und/oder Teilbereiche einer Verklebung mit Referenzwerten der geometrischen Dimensionen beurteilt wird.
42. Verfahren nach einer der Ausführungsformen 33 bis 39, wobei die Auswertung die Bildung eines Integrals über einen zeilenförmigen Teilbereich der thermografischen Daten einer oder mehrerer isothermer Bereiche und/oder Teilbereiche in x-Richtung umfasst.
43. Verfahren nach Ausführungsform 42, wobei die Verklebegüte anhand eines Vergleichs des Integrals des einen oder der mehreren isothermen Bereiche und/oder Teilbereiche einer Verklebung mit Referenzwerten des jeweiligen Integrals beurteilt wird.
44. Verfahren nach einer der Ausführungsformen 33 bis 39, wobei die Auswertung die Bildung eines Flächenintegrals über einen Teilbereich der thermografischen Daten einer oder mehrerer isothermer Bereiche und/oder Teilbereiche in x-Richtung und y-Richtung umfasst.
45. Verfahren nach Ausführungsform 44, wobei die Verklebegüte anhand eines Vergleichs des Flächenintegrals des einen oder der mehreren isothermen Bereiche und/oder Teilbereiche einer Verklebung mit Referenzwerten des jeweiligen Flächenintegrals beurteilt wird.
46. Verfahren nach einer der Ausführungsformen 41, 43 oder 45, wobei die Referenzwerte, vorbestimmte Referenzwerte umfassen und/oder über einen Durchschnitt der vorherig überprüften Verklebungen gebildet werden, insbesondere der zwei vorherig überprüften Verklebungen, bevorzugt der fünf vorherig überprüften Verklebungen und besonders bevorzugt der mindestens zehn vorherig überprüften Verklebungen.
47. Verfahren nach einer der Ausführungsformen 41, 43, 45 oder 46, wobei die Verklebegüte ausreichend ist, wenn der jeweilige Vergleich eine maximale Abweichung von 0% bis 50%, bevorzugt von 0% bis 20% und besonders bevorzugt von 0% bis 5% ergibt.
48. Verfahren nach einer der vorangehenden Ausführungsformen 33 bis 47, wobei das Detektieren der thermischen Strahlung mit einer Infrarot-Kamera durchgeführt wird.
49. Verfahren nach einer der Ausführungsformen 33 bis 48, wobei das Verfahren in-line beim Herstellungsprozess von Papierverklebungen, vorzugsweise Wellpappenverklebungen durchgeführt wird.
50. Verfahren nach einer der Ausführungsformen 33 bis 49, wobei die thermische Strahlung von einer Oberflächenseite der jeweils ersten und/oder der mindestens zweiten Papierbahn detektiert wird, deren Normalenvektor in die entgegengesetzte Richtung der jeweils anderen Papierbahn gerichtet ist.
51. Verfahren nach einer der Ausführungsformen 33 bis 50, wobei die Papierverklebung eine Wellpappenverklebung ist.
52. Verfahren nach einer der Ausführungsformen 33 bis 51, wobei die Produktbahn eine Wellpappenbahn ist.
53. Verfahren nach einer der Ausführungsformen 33 bis 52, wobei das Verfahren mit einem Messsystem gemäß einer der Ausführungsformen 1 bis 28 durchgeführt wird.

## Patentansprüche

1. Messsystem (1) zur Verwendung in einer Wellpappenmaschine (2), wobei das Messsystem (1) eine Messeinrichtung (10) umfasst;
**dadurch gekennzeichnet, dass**
die Messeinrichtung (10) elektromagnetische Wellen im Infrarot-Bereich erfasst und auf einen Bereich einer Transporteinrichtung (27) der Wellpappenmaschine (2) in Transportrichtung (94a) hinter einer Klebestation (22) der Wellpappenmaschine (2) gerichtet ist.

2. Messsystem (1) nach Anspruch 1, **dadurch gekennzeichnet, dass** die Messeinrichtung (10) ausgelegt ist, die Temperatur, vorzugsweise die Oberflächentemperatur der Wellpappe (28) zu erfassen, und optional eine Temperaturverteilung (40a, 42a) thermografisch darzustellen und zu analysieren.

3. Messsystem nach irgendeinem der vorangehenden Ansprüche, **dadurch gekennzeichnet, dass** die Messeinrichtung eine Infrarot-Kamera und/oder eine bildgebende Messeinrichtung umfasst.

4. Messsystem (1) nach irgendeinem der vorangehenden Ansprüche, **dadurch gekennzeichnet, dass** die Messeinrichtung (10) ausgelegt ist eine Temperaturverteilung (40a, 42a) der Wellpappe (28), vorzugsweise eine Temperaturverteilung (40a, 42a) der Wellpappe (28) entlang der Bahnbreite (92a) zu erfassen.

5. Messsystem (1) nach irgendeinem der vorangehenden Ansprüche, **dadurch gekennzeichnet, dass** die Messeinrichtung (10) eine Auswerteeinrichtung (14) zur Auswertung der erfassten elektromagnetischen Wellen im Infrarot-Bereich umfasst.

6. Messsystem (1) nach Anspruch 5, **dadurch gekennzeichnet, dass** die Auswerteeinrichtung (14) einen Computer mit einer Speichereinrichtung umfasst, wobei auf der Speichereinrichtung ein Computerprogramm zur Überprüfung des Herstellungsprozesses von Verklebungen einer ersten Papierbahn (24) mit mindestens einer zweiten Papierbahn (25, 26) zu einer Produktbahn (28) gespeichert ist, das ausgelegt ist, die folgenden Schritte auszuführen:
- Detektieren thermischer Strahlung der Produktbahn (28) nach dem Verkleben der ersten Papierbahn (24) mit der mindestens zweiten Papierbahn (25, 26),
- Erstellen thermografischer Daten (30) basierend auf der thermischen Strahlung,
- Auswerten der thermografischen Daten (30), und
- Beurteilen der Verklebegüte der Papierverklebung anhand der Auswertung.

7. Messsystem (1) nach Anspruch 6, wobei die thermische Strahlung der Produktbahn (28) in einem Erfassungsbereich (31), der sich zeilenförmig (31b) in y-Richtung (92) der Produktbahn (28) und/oder zweidimensional (31a) in x- und y-Richtung (94, 92) der Produktbahn (28) erstreckt, detektiert wird.

8. Messsystem (1) nach irgendeinem der vorangehenden Ansprüche 6 oder 7, wobei die Beurteilung der Verklebegüte anhand einer geometrischen Auswertung der thermografischen Daten (30) erfolgt.

9. Messsystem (1) nach irgendeinem der vorangehenden Ansprüche 6 bis 8, wobei die Auswertung eine ortsaufgelöste Bestimmung der geometrischen Dimensionen, bevorzugt der Länge (44a, 46a) in x-Richtung (94) und/oder der Breite in y-Richtung (92), einer oder mehrerer isothermer Bereiche (32a) und/oder Teilbereiche in den thermografischen Daten (30) umfasst.

10. Messsystem (1) nach irgendeinem der vorangehenden Ansprüche 6 bis 9, wobei die Auswertung die Bildung eines Integrals (48) und/oder Flächenintegrals über einen zeilenförmigen Teilbereich der thermografischen Daten (30) einer oder mehrerer isothermer Bereiche (32a) und/oder Teilbereiche in x-Richtung (94) umfasst.

11. Messsystem (1) nach irgendeinem der vorangehenden Ansprüche 9 oder 10, wobei die Verklebegüte anhand eines Vergleichs der geometrischen Dimensionen (44, 46) und/oder eines Vergleichs des Integrals (48) und/oder eines Vergleichs des Flächenintegrals des einen oder der mehreren isothermen Bereiche (32a) und/oder Teilbereiche einer Verklebung mit Referenzwerten der geometrischen Dimensionen (44, 46) beurteilt wird, und optional, wobei die Referenzwerte, vorbestimmte Referenzwerte umfassen und/oder über einen Durchschnitt der vorherig überprüften Verklebungen gebildet werden, insbesondere der zwei vorherig überprüften Verklebungen, bevorzugt der fünf vorherig überprüften Verklebungen und besonders bevorzugt der mindestens zehn vorherig überprüften Verklebungen.

12. Wellpappenmaschine (2) umfassend:
eine Transporteinrichtung (27) zum Transport von Wellpappe (28) in einer Transportrichtung (94a),
eine Klebestation (22) ausgelegt zum Verkleben einer ersten Papierbahn (24) mit mindestens einer zweiten Papierbahn (25, 26), und
ein Messsystem zur Überprüfung des Herstellungsprozesses von Verklebungen der Wellpappe (28);
**dadurch gekennzeichnet, dass**
das Messsystem ein Messsystem (1) nach einem der Ansprüche 1 bis 11 ist.

13. Verfahren zur Überprüfung des Herstellungsprozesses von Verklebungen einer ersten Papierbahn (24) mit mindestens einer zweiten Papierbahn (25, 26) zu einer Produktbahn (28), unter Verwendung eines Messsystems,
**gekennzeichnet durch** die folgenden Schritte:
- Detektieren thermischer Strahlung der Produktbahn (28) nach dem Verkleben der ersten Papierbahn (24) mit der mindestens zweiten Papierbahn (25, 26),
- Erstellen thermografischer Daten (30) basierend auf der thermischen Strahlung,
- Auswerten der thermografischen Daten (30), und
- Beurteilen der Verklebegüte der Papierverklebung anhand der Auswertung.

14. Verfahren nach Anspruch 13, wobei die thermische Strahlung von einer Oberflächenseite der jeweils ersten und/oder der mindestens zweiten Papierbahn detektiert wird, deren Normalenvektor in die entgegengesetzte Richtung der jeweils anderen Papierbahn gerichtet ist.

15. Verfahren nach einem der Ansprüche 13 oder 14, wobei das Verfahren mit einem Messsystem (1) gemäß einem der Ansprüche 1 bis 11 durchgeführt wird.
